# EUROPÄISCHE PATENTSCHRIFT

(11) **EP 3 079 737 B1**
(45) Veröffentlichungstag und Bekanntmachung des Hinweises auf die Patenterteilung: **04.07.2018**
(21) Anmeldenummer: 14824363.7
(22) Anmeldetag: 10.12.2014
(51) Int. Cl.: A61M 1/36, A61F 7/00

(54) **KÜHLEINHEIT FÜR EINEN WÄRMETAUSCHER ZUR TEMPERIERUNG VON IN EINEM EXTRAKORPORALEN BLUTKREISLAUF GEFÜHRTEM BLUT**
COOLING UNIT FOR A HEAT EXCHANGER FOR CONTROLLING THE TEMPERATURE OF BLOOD CONDUCTED IN AN EXTRACORPOREAL CIRCUIT
UNITÉ DE REFROIDISSEMENT POUR ÉCHANGEUR DE CHALEUR DESTINÉ À ÉQUILIBRER LA TEMPÉRATURE DU SANG CIRCULANT DANS UN CIRCUIT SANGUIN EXTRACORPOREL

(30) Priorität: 10.12.2013 DE 202013011109 U
(43) Veröffentlichungstag der Anmeldung: 19.10.2016
(73) Patentinhaber: ResuSciTec GmbH, 79098 Freiburg (DE)
(72) Erfinder: WILKE, Joachim, 73660 Urbach (DE); BENK, Christoph, 79106 Freiburg (DE); PITTNER, Martin, 73035 Göppingen (DE); KAZ, Till, 70186 Stuttgart (DE)
(74) Vertreter: Rösler, Uwe
(86) Internationale Anmeldenummer: PCT/EP2014/003307
(87) Internationale Veröffentlichungsnummer: WO 2015/086147

(56) Entgegenhaltungen:
- WO-A2-2009/009211
- DE-A1-102005 007 516
- US-A1- 2007 244 475
- US-A1- 2011 146 939

## Beschreibung

### Technisches Gebiet

Die Erfindung bezieht sich auf eine Kühleinheit für einen in einem Oxygenator integrierten Wärmetauscher zur Temperierung von in einem extrakorporalen Blutkreislauf geführtem Blut, mit einem ersten Behältnis, das zumindest teilweise mit einem flüssigen Arbeitsmittel befüllt ist, das in thermischen Kontakt mit einer in einem Schlauchleitungssystem geführten Wärmeträgerflüssigkeit des Wärmetauschers steht.

### Stand der Technik

Im Rahmen der Herzchirurgie oder Intensivmedizin insbesondere zur Behandlung von akutem Lungenversagen finden Herz-Lungen-Maschinen Anwendung , mit denen die Pumpfunktion des Herzens sowie die Lungenfunktion für einen beschränkten Zeitraum ersetzbar sind. Das Blut verlässt dabei über einen extrakorporalen Blutkreislauf in Form eines Schlauchsystems den Körper, wird mit Hilfe eines Oxygenators, der Teil der Herz-Lungenmaschine ist, mit Sauerstoff angereichert und wieder in den Körper zurückgeführt. Der Oxygenator übernimmt dabei die Funktion der Lungen und führt dem Blut nicht nur den lebenswichtigen Sauerstoff zu, sondern entfernt gleichzeitig das durch Stoffwechselprozesse entstehende Kohlendioxid (CO2).

Alle heute verwendeten Oxygenatoren besitzen zusätzlich einen Wärmetauscher, mit dessen Hilfe sich das durchströmende Blut erwärmen oder abkühlen lässt. So finden die Eingriffe am Herzen in der Regel unter Hypothermiebedingungen statt, d.h. das Blut wird mehr oder weniger stark abgekühlt, wodurch die Stoffwechselaktivität und der Sauerstoffverbrauch im Körper des Patienten reduziert wird.

Beispiele zur Ausbildung gängiger Wärmetauscher sind in den Druckschriften DE 10 2010 000 820 A1, DE 10 2012 204 705 A1 oder WO 2011/139392 A1 beschrieben. Allen modernen Wärmetauschern ist gemein, dass durch eine Vielzahl einzelner, parallel geführter Schläuche das extrakorporal geführte Blut mit jeweils einheitlicher Strömungsrichtung druckbeaufschlagt geleitet wird, wobei das Schlauchbündel in Gegen- oder Querstromrichtung zur Blutströmung in thermischen Kontakt mit einer Wärmeträgerflüssigkeit tritt, typischerweise in Form einer das Schlauchbündel umströmenden Wasserströmung.

Zur Abkühlung der durch den Wärmetauscher strömenden und mit dem Blut in thermischen Kontakt tretenden Wärmeträgerflüssigkeit dient ein so genanntes Hypothermiegerät, das zumeist als portable Einheit ausgebildet und ein von Wasseranschlüssen unabhängiges Kühlgerät darstellt, das über Anschlüsse an den im Oxygenator integrierten Wärmetauscher fluiddicht anschließbar ist.

In modernen Hypothermiegeräten wird die Erzeugung von Kälte durch die so genannte Siedekühlung realisiert. Dabei entsteht der Kühleffekt durch die Verdampfungsenergie, die aufgebracht werden muss, um ein Kältemittel von einem flüssigen in einen gasförmigen Aggregatszustand zu überführen. Diese nötige Verdampfungsenergie wird der Umgebung bzw. dem Wasser in Form von Wärme entzogen und führt somit zu dem gewünschten Kühleffekt.
Die Siedekühlung gilt als eine der effektivsten Möglichkeiten, einem System Wärme bzw. thermische Energie zu entziehen. Hypothermiegeräte, die diese Technologie nutzen, werden der Klasse der Kompressionskältemaschinen zugeordnet, in denen ein Kältemittel in einem geschlossenen Kreislauf geführt wird und nacheinander verschiedene Aggregatzustandsänderungen erfährt. So wird gasförmiges Kältemittel durch einen Kompressor komprimiert, in einem nachfolgenden Wärmeübertrager kondensiert und verflüssigt, wobei das Kältemittel Wärme abgibt. Anschließend wird das flüssige Kältemittel aufgrund einer Druckänderung, zum Beispiel mittels eines Expansionsventil oder eines Kapillarrohrs entspannt. In einem nachgeschalteten zweiten Wärmeübertrager, bspw. in Form eines Verdampfers, verdampft das Kältemittel unter Wärmeaufnahme bei niedriger Temperatur, die so genannte Siedekühlung. Der Kreislauf kann nun von vorne beginnen. Der Prozess erfordert jedoch die Zufuhr von Antriebsenergie, um den Kompressor in Gang zu halten.

Handelsübliche Hypothermiegeräte sind großbauende und schwergewichtige, zumeist auf Rollen gelagerte Bedieneineinheiten, deren Heiz- und Kühlaggregate über wenigstens ein Netzkabel mit elektrischer Energie versorgt werden. Über ebenfalls geräteseitig vorgesehene Anschlussstutzen, über die das Fluidleitungssystem des im Oxygenator integrierten Wärmetauschers anschließbar ist, gelangt die Wärmeträgerflüssigkeit des Wärmetauschers in das Hypothermiegerät, in dem sie vorzugsweise gekühlt wird.

Der DE 20 2004 001 194 U1 ist ein vorstehend beschriebenes Hypothermiegerät zu entnehmen, das zur Reinhaltung des als Kältemittel dienenden flüssigen Arbeitsmittels, hier in Form von Wasser, UV-Lampen vorsieht, deren UV-Strahlung durch UV-Licht transparente Schläuche hindurch das in den Schläuchen geführte Wasser entkeimt.

Ein anderes Prinzip zur Kälteerzeugung machen sich Sorptionsmittel gestützte Kühlsysteme zu Nutze, die für eine zeitlich begrenzte Dauer Kühlleistung kurzfristig zu generieren vermögen ohne die grundsätzliche Notwendigkeit elektrische Energie zu benötigen. In einem unter Unterdruckbedingungen befindlichen Aufnahmevolumen ist ein Sorptionsmittel, bspw. Zeolith, enthalten, das in der Lage ist ein Kühlmittel, bspw. in Form von Wasserdampf, zu ab- oder adsorbieren. Das Kühlmittel wird in einem separaten, sogenannten Verdampfervolumen in flüssiger Form vorzugsweise unter Dampfdruckbedingungen bevorratet. Das Aufnahmevolumen ist über eine kontrolliert auslösbare Trenneinrichtung fluidisch mit dem Verdampfervolumen verbindbar, so dass im Falle einer fluidischen Anbindung der Druck im Verdampfervolumen schlagartig aufgrund des Druckausgleiches mit dem Aufnahmevolumen unter den Dampfdruck des Kältemittels absinkt, wodurch dieses verdampft und der Umgebung Wärme entzieht. Der Kühlmitteldampf wird vom Sorptionsmittel innerhalb des Aufnahmevolumens ab- bzw. adsorbiert und gibt dabei die im Wege der Verdampfung freiwerdende Verdampfungswärme an das Sorptionsmittel ab.

Das vorstehende Kühlprinzip macht sich eine Hypothermievorrichtung für den menschlichen und tierischen Körper zunutze, die in der Druckschrift DE 20 2012 003 544 U1 offenbart ist. Die vorstehend erläuterten Aufnahme- und Verdampfervolumina sind als flexibles Kühlelement ausgebildet, das zum direkten Auflegen am Körper ausgebildet ist. Hierbei umschließt eine Folie, vorzugsweise eine Mehrschichtfolie die einzelnen Volumina, die über eine thermische Isolationsschicht voneinander getrennt angeordnet sind.

Die Druckschrift US 2008/0255644 A1 beschreibt eine vergleichbare Kühleinrichtung zur vorstehenden Hypothermievorrichtung. Ein mit Wasser gefülltes Volumen, das flächig an ein wärmeleitfähiges Kissenelement zur direkten Auflage an einen zu kühlenden Körper angrenzt, ist über ein ventilartig ausgebildetes Verbindungsstück rückseitig zum Kissenelement mit einem ein Sorptionsmittel enthaltendes Aufnahmevolumen fluidisch verbindbar.

Die Druckschrift US 2007/0244475 A1 offenbart eine Vorrichtung zum Kühlen einer in einem flexiblen Vorratsbeutel bevorrateten Flüssigkeit zur unmittelbaren Infusion in einen menschlichen oder tierischen Körper. Die Vorrichtung verfügt über eine flächig ausgebildete Kühleinheit, die abnehmbar fest mit einem Sorptionsmittel-Wärmetauscher thermisch gekoppelt ist, in dem getrennt von einem Sorptionsmittelvolumen ein Flüssigkeitsreservoir untergebracht ist. Mittels kontrollierter Perforation des Flüssigkeitsreservoirs ergießt sich die Flüssigkeit kontrolliert in ein evakuiertes Verdampfervolumen, das einerseits über eine dampfdurchlässige Membran unmittelbar an das Sorptionsmittelvolumen und andererseits an die Kühleinheit flächig angrenzt und thermisch an diese ankoppelt. Das sich im Wege der Absorption erwärmende Sorptionsmittel ist somit einerseits vom Verdampfervolumen und andererseits von Flüssigkeitsreservoir begrenzt.

Die Druckschrift WO 2009/009211 beschreibt einen kühlbaren Motorradanzug, der mit Kühlluft beaufschlagt werden kann. In der in dieser Druckschrift beschriebenen Figur 1 werden ein erstes Behältnis, in dem Wasser bevorratet ist, und ein zweites Behältnis offenbart, in dem ein Sorptionsmittel enthalten ist. Beide Behältnisse sind über eine Verbindungsleitung verbunden, längs der ein Ventil eingebracht ist. Durch Öffnen des Ventils strömt Wasser in das zweite Behältnis, wodurch das im ersten Behältnis befindliche Wasser entwärmt, d. h. gekühlt wird. Um das erste Behältnis wird ein Luftstrom geführt, angetrieben über ein Gebläse, der über eine entsprechende Verbindungsstelle dem nicht weiter dargestellten Motorradanzug zugeführt wird.

Die Druckschrift US 2011/0146939 A1 beschreibt eine Energie absorbierendes sowie abgebendes System, das einen mit Zeolith gefüllten Container aufweist, in den ein Flächenelement hineinragt, das in thermischen Kontakt mit einer Wärmequelle, bspw. eine Solarzelle steht. Über entsprechende Verbindungsleitungen wird Wasser in den Container zur Aktivierung des Zeoltih geleitet.

### Darstellung der Erfindung

Der Erfindung liegt die Aufgabe zugrunde, eine Kühleinheit für einen in einem Oxygenator integrierten Wärmetauscher, der zur Behandlung von einem in einem extrakorporalen Blutkreislauf geführtem Blut dient, möglichst klein und leichtgewichtig auszubilden, um einen manuell portablen Einsatz, insbesondere geeignet für die Notfallmedizin, zu ermöglichen. In besonderer Weise sollte die Kühleinheit vollkommen autark, d. h. unabhängig von einer externen Stromversorgung, betreibbar sein. Die Kühlleistung der Kühleinheit soll jedoch vergleichbar sein mit handelsüblichen Hypothermiegeräten, die mittels elektrischer Energiezufuhr auf dem Prinzip der eingangs erläuterten Siedekühlung arbeiten und im Einsatz mit Oxygenatoren betrieben werden. Insbesondere gilt es eine Kühlleistung zu generieren, mit der es möglich sein soll, das Blut im menschlichen Blutkreislauf mittels des Oxygenators innerhalb einer Zeitspanne von ca. 30 Minuten um wenigstens 4K abzukühlen. Darüber hinaus sollen Vorkehrungen getroffen werden, um den Kühlbetrieb kontinuierlich und dauerhaft fortführen zu können.

Die Lösung der der Erfindung zugrunde liegenden Aufgabe ist im Anspruch 1 angegeben. Die lösungsgemäße Kühleinheit in vorteilhafter Weise ausbildende Merkmale sind Gegenstand der Unteransprüche sowie der weiteren Beschreibung insbesondere unter Bezugnahme auf das illustrierte Ausführungsbeispiel zu entnehmen.

Die Kühleinheit für einen in einem Oxygenator integrierten Wärmetauscher zur Temperierung von in einem extrakorporalen Blutkreislauf geführtem Blut, verfügt über ein erstes Behältnis, das zumindest teilweise mit einem flüssigen Arbeitsmittel befüllt ist, das im thermischen Kontakt mit einer vorzugsweise in einem Schlauchleitungssystem geführten Wärmeträgerflüssigkeit des Wärmetauschers steht. Ferner ist ein zweites Behältnis vorgesehen, das ein Sorptionsmittel unter Vakuumbedingungen fluiddicht umschließt und über wenigstens einen Kontaktabschnitt an das erste Behältnis angrenzt. Am oder innerhalb des Kontaktbereiches ist ein Trennmittel angebracht, das bei Betätigung eine lokal begrenzte fluidische Verbindung zwischen dem ersten zum zweiten Behältnis herstellt, wodurch das flüssige Arbeitsmittel, vorzugsweise in Form von Wasser, aufgrund des zwischen dem ersten und zweiten Behältnis bestehenden Druckunterschiedes in das zweite Behältnis entweicht, wobei die in das zweite Behältnis entweichenden Anteile des flüssigen Arbeitsmittels verdampfen und einen Arbeitsmitteldampf bilden, der von dem innerhalb des zweiten Behältnisses enthaltenen Sorptionsmittel sorbiert wird. Zugleich entnimmt das verdampfende Arbeitsmittel dem im ersten Behältnis enthaltenen flüssigen Arbeitsmittel Wärme, die im Rahmen des zweiten Behältnisses an das Sorptionsmittel in Form von Verdampfungswärme abgegeben wird. Das erste und zweite Behältnis sind von einer thermisch isolierenden Umfassung umgeben, wobei das erste und zweite Behältnis zusammen mit dem Kontaktabschnitt jeweils ein Volumen hermetisch umschliessen, und das erste Behältnis wenigstens teilweise in ein Bad mit der Wärmeträgerflüssigkeit eintaucht. Das erste und zweite Behältnis sind dabei jeweils als doppelwandige Hohlzylinder ausgebildet und umschließen jeweils ein Ringvolumen. Beide doppelwandig ausgebildete Hohlzylinder sind längs ihrer den beiden Hohlzylindern zugeordneten Zylinderachsen einander stirnseitig zugewandt über den gemeinsamen Kontaktabschnitt verbunden und bilden eine modulare, einheitlich handhabbare kühlende Funktionseinheit.

Die lösungsgemäße Kühleinheit nutzt gleichsam wie gattungsgemäße Hypothermiegeräte, das Prinzip der Siedekühlung, verzichtet jedoch auf einen mit elektrischer Energie zu versorgenden Kompressor und nutzt vielmehr eine ausschließlich druckgetriebene Verdampfung von Wasser als Arbeitsmittel, das innerhalb des ersten Behälters, vorzugsweise bei einem Druck bevorratet ist, der knapp unter dem Dampfdruck von Wasser liegt, so dass das Wasser im ersten Behältnis in flüssiger Form vorliegt. Wird der zwischen dem ersten und dem zweiten Behälter befindliche Kontaktabschnitt, der beide Behälter gegenseitig fluiddicht trennt, mit Hilfe einer vorzugsweise manuell bedienbaren Trennvorrichtung, bspw. in Form einer Schneidevorrichtung, durchstoßen, so sinkt der im ersten Behältnis vorherrschende Druck schlagartig ab. Im Wege eines lokalen Druckausgleiches, zumindest im Bereich des Kontaktabschnittes sinkt der Druck unter den Dampfdruck des Wassers ab, wodurch dieses anteilig unter Freisetzung von Verdampfungswärme verdampft, die dem übrigen, im ersten Behältnis verbleibenden Wassers entzogen wird, wodurch dieses signifikant abgekühlt wird.

In einer bevorzugten Ausführungsform befinden sich innerhalb des zweiten Behältnisses Zeolithe in Form synthetischer oder natürlicher Zeolithe, die den sich ausbildenden Wasserdampf zu adsorbieren vermögen. Aufgrund der zeitlichen Trägheit mit der das im zweiten Behältnis bevorratete Sorptionsmittel, das auch aus anderen Sorptionsmaterialien, wie bspw. Siliziumverbindungen, bestehen kann, den sich ausbildenden Wasserdampf zu absorbieren vermag, stellt sich nicht sofort ein Reaktionsgleichgewicht ein, wodurch das Druckgefälle zwischen dem ersten und zweiten Behältnis für eine begrenzte Zeit erhalten bleibt, so dass ein weiteres Verdampfen von im ersten Behältnis enthaltenen Wassers ermöglicht wird.

Die Form sowie auch Größe des ersten und zweiten Behältnisses sowie die innerhalb des ersten Behältnisses bevorratete Arbeitsmittelmenge, vorzugsweise in Form von Wasser, sowie die Menge und Lagerung des im zweiten Behältnisses bevorrateten Sorptionsmittels, sind in geeigneter Weise derart aufeinander abgestimmt, so dass durch sukzessives Verdampfen des innerhalb des ersten Behältnisses bevorrateten Arbeitsmittels eine zeitlich ausgedehnte Abkühlung des innerhalb des ersten Behälters bevorrateten Arbeitsmittels ermöglicht wird, ohne dabei den Kühlprozess durch externe Stromversorgung zu unterstützen.

Zur technischen Nutzung der Kühleinheit, die auf der Grundlage der vorstehend erläuterten, stromlosen Siedekühlung basiert, befindet sich das innerhalb des ersten Behälters enthaltene flüssige Arbeitsmittel in thermischem Kontakt mit einem Schlauchleitungssystem, durch das die Wärmeträgerflüssigkeit eines innerhalb eines Oxygenators integrierten Wärmetauschers mit Hilfe einer längs des Schlauchleitungssystems angeordneten Förderpumpe geleitet wird. Auch sind die Ausbildung und der Einsatz der Kühleinheit für einen Wärmetauscher denkbar, der unabhängig von einem Oxygenator in Form einer eigenständigen Funktionseinheit ausgebildet und längs eines extrakorporalen Blutleitungsweges, bspw. längs eines Kardioplegiekreislaufes, an einer geeigneten Stelle eingebracht ist. Die weitere Beschreibung bezieht sich jedoch ohne Einschränkung auf den allgemeinen Lösungsgedankens auf eine Wärmtauschereinheit als integralen Bestandteil eines Oxygenators.

In einer bevorzugten Ausführungsform ist die lösungsgemäße Kühleinheit als einheitliches Gerätemodul ausgebildet, das vorzugsweise über standardisierte fluiddichte Schlauchleitungskonnektoren mit dem Schlauchleitungssystem eines in einem handelsüblichen Oxygenator integrierten Wärmetauschers fluiddicht anschließbar ist, d.h. Wärmetauscherseitig bedarf es keinerlei Modifikationen, um die lösungsgemäße Kühleinheit in Betrieb zu nehmen. Die lösungsgemäße Kühleinheit verfügt hierzu über wenigstens einen Schlauchleitungsabschnitt der zumindest bereichsweise in mittelbaren oder unmittelbaren thermischen Kontakt mit dem im ersten Behälter bevorrateten flüssigen Arbeitsmittel steht. Der wenigstens eine Schlauchleitungsabschnitt verfügt darüber hinaus über eine Zu- und Ableitungsöffnung, die außerhalb des ersten Behälters angeordnet sind. An der Zu- und Ableitungsöffnung sind vorzugsweise handelsübliche Schlauchleitungskonnektoren, bspw. in Form von Luer-Lock-Anschlüssen oder einer Hansen-Kupplung, angebracht, die mit am Schlauchleitungssystem des Wärmetauschers vorgesehenen Leitungsanschlüssen fluiddicht lösbar verbindbar sind.

Für eine möglichst effektive Kühlung der Wärmeträgerflüssigkeit, die durch das Wärmetauscher eigene Schlauchleitungssystem und den damit zu einem geschlossenen Strömungskreislauf verbundenen, zur lösungsgemäßen Kühleinheit zugehörigen Schlauchleitungsabschnitt geleitet wird, verläuft der mit dem flüssigen Arbeitsmittel in thermischem Kontakt stehende Bereich des Schlauchleitungsabschnittes durch den ersten Behälter, so dass ein direkter Wärmeübergang von dem flüssigen Arbeitsmittel durch die Wand des Schlauchleitungsabschnittes auf die Wärmeträgerflüssigkeit erfolgen kann. Hierzu durchragt der im Inneren des ersten Behälters mit dem flüssigen Arbeitsmittel in unmittelbarem thermischen Kontakt stehende Bereich des Schlauchleitungsabschnittes die den ersten Behälter fluiddicht umgebende Behälterwand nach Außen für den fluiddichten Anschluss an das Schlauchleitungssystem des Wärmetauschers.

Alternativ oder in Kombination ist es möglich, durch geeignete Wahl der den ersten Behälter fluiddicht umgebenden Behälterwand den mit dem flüssigen Arbeitsmittel in thermischen Kontakt tretenden Bereich des Schlauchleitungsabschnittes unmittelbar an der Behälterwand anzubringen oder in diese zu integrieren.

Nicht notwendigerweise ist es erforderlich, die jeweils den ersten und zweiten Behälter fluiddicht umgebenden Behälterwände aus einem starren, vorzugsweise metallischen Wandmaterial zu fertigen, gleichwohl ist es möglich die Behälterwandmaterialien flexibel und dünnwandig auszugestalten. Im letzteren Fall ist jedoch dafür Sorge zu tragen, dass zumindest innerhalb des zweiten Behälters aufgrund der dort vorherrschenden Vakuumdruckbedingungen innere, die flexible Behälterwand gegen Kompressionseffekte wirkende Stützstrukturen vorzusehen sind, um freie Ausbreitungswege innerhalb des zweiten Behältnisses für das verdampfte Arbeitsmittel zu schaffen, das von dem Sorptionsmittel sorbiert wird.

Zur Gewährleistung einer Wärmeträgerflüssigkeitsströmung durch den geschlossenen Strömungskreislauf, der sich aus dem Wärmetauscher eigenen Schlauchleitungssystem und dem der Kühlereinheit zuordenbaren Schlauchleitungsabschnitt zusammensetzt, ist längs des Schlauchleitungsabschnittes eine Förderpumpe integriert. Die Förderpumpe befindet sich vorzugsweise in fluiddicht gekapselter Form innerhalb des ersten Behälters und ist darüber hinaus, um dem geforderten Aspekt einer Stromversorgungsunabhängigen Betriebweise Rechnung zu tragen, mit einer Batterieeinheit kombiniert, die eine ansonsten autarke Betriebsweise der Förderpumpe ermöglicht. Selbstverständlich ist es ebenfalls möglich, die Förderpumpe auch außerhalb des ersten Behälters längs des Schlauchleitungsabschnittes vorzusehen.

Sowohl aus Gründen der Realisierung eines möglichst kompakten äußeren Erscheinungsbildes und vor allem auch aus thermischen Aspekten sind der erste und zweite Behälter von einer thermischen Umfassung bzw. von einem Thermogehäuse umgeben, in die bzw. in das beide Behälter modular einsetzbar bzw. herausnehmbar gelagert sind.

Eine typische Baugröße für die thermische Umfassung bemisst sich nach Breite, Höhe und Tiefe in der Größenordnung von 200 x 290 x 440 mm. Ein geeignetes Volumen zur Aufnahme des flüssigen Arbeitsmittels innerhalb des ersten Behälters bemisst sich typischerweise zwischen 0,5 l und 2 l, vorzugsweise zwischen 0,5 l und 1 Liter.

Die lösungsgemäße Kühleinheit eignet sich somit in Kombination mit einem handelsüblichen Oxygenator, in dem ein Wärmetauscher integriert ist, dessen Wärmeträgerflüssigkeit mit konstruktiv einfachem und energetisch unabhängigen Mitteln gekühlt werden kann. Die Kühleinheit richtet sich vornehmlich für jene Einsätze, in denen gleichfalls portable Oxygenatoren und ebenfalls damit verbundene, portabel ausgebildete medizinische Geräte zur Realisierung extrakorporaler Blutkreisläufe zum Einsatz kommen. In besonders vorteilhafter Weise eignet sich die lösungsgemäße Kühleinheit zur Kühlung der Wärmeträgerflüssigkeit eines Wärmetauschers, der innerhalb des extrakorporalen Blutkreislaufes einer Vorrichtung integriert ist, zur Behandlung eines Individuums mit Herz- oder Kreislaufstillstand, die ein am Individuum applizierbares Blutentnahmemittel zur Entnahme zumindest eines Teils des Blutes aus dem Individuum vorsieht. Die Vorrichtung verfügt ferner über eine mittel- oder unmittelbar mit dem Blutentnahmemittel verbundene Analyseeinheit zur Erfassung und Bereitstellung wenigstens einer Eigenschaft des Blutes in Form eines Blutanalyseergebnisses, eine Wirkeinheit, die mittel- oder unmittelbar mit einem am Individuum applizierbaren Rückführungsmittel verbunden ist und zur Abgabe einer Substanz über das Rückführungsmittel an das Individuum ausgebildet ist, wobei die Wirkeinheit wenigstens eine Reservoireinheit umfasst, die wenigstens zwei Substanzen bevorratet, wobei einer mit der Reservoireinheit kombinierte Dosiereinheit vorgesehen ist, die zumindest unter Berücksichtigung eines von der Analyseeinheit ermittelten Blutanalyseergebnisses aus den wenigstens zwei Substanzen wenigsten eine Substanz ausfüllt oder eine Mischung aus den wenigstens zwei der Substanzen anfertigt und wobei schließlich die wenigstens eine ausgewählte Substanz oder die Mischung mittel- oder unmittelbar über das Rückführungsmittel in das Individuum einbringbar ist. Eine derartige Vorrichtung ist insbesondere der EP 2 488 231 A1 zu entnehmen.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigt:
- Fig. 1: schematische Darstellung einer beispielhaften, nicht erfindungsgemäßen Kühleinheit mit einem an sich bekannten Oxygenator.
- Fig. 2: schematisierte Darstellung einer erfindungsgemäßen, modular ausgebildeten Kühleinheit mit verbesserten Kühleigenschaften,
- Fig. 3a, b: Ausführungsbeispiel für eine erfindungsgemäße Kühleinheit mit verbesserten Kühleigenschaften sowie
- Fig. 4: Längsteilschnittdarstellung durch gemeinsamen Kontaktabschnitt eines ersten und zweiten Behältnisses einer erfindungsgemäßen Kühleinheit mit verbesserten Kühleigenschaften.

### Wege zur Ausführung der Erfindung, gewerbliche Verwendbarkeit

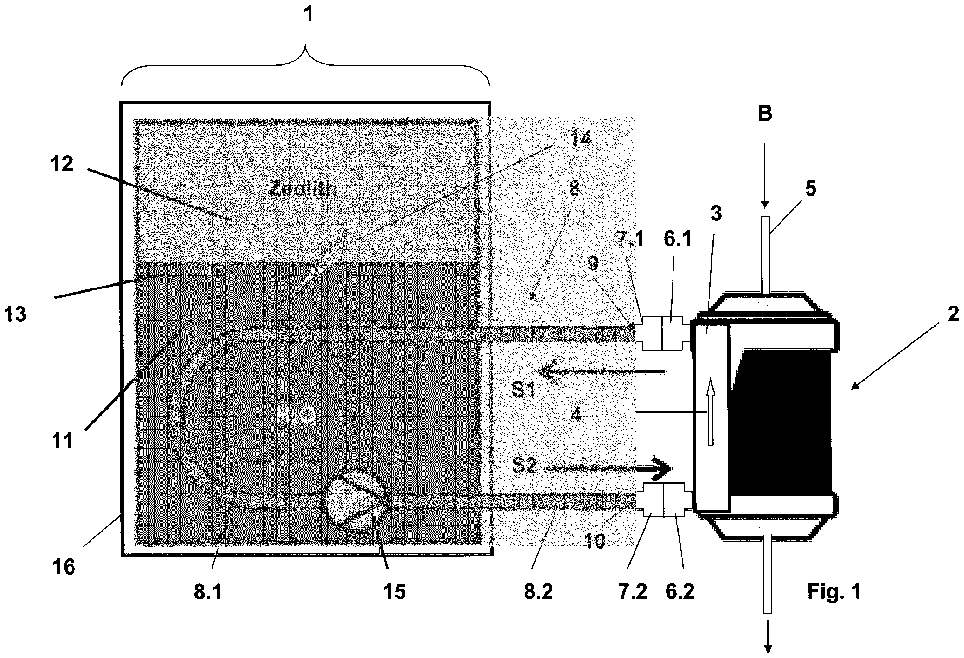

Figur 1 stellt ein Beispiel einer nicht erfindungsgemäßen Kühleinheit 1 mit einem an sich bekannten Oxygenator 2 dar. Es sei angenommen, dass innerhalb des Oxygenators 2 ein Wärmetauscher 3 integriert ist, der über ein Schlauchleitungssystem 4 verfügt, durch das die Wärmeträgerflüssigkeit des Wärmetauschers fließt, die in thermischen Kontakt mit dem durch den Oxygenator 2 längs eines nicht weiter dargestellten extrakorporalen Blutkreislaufes 5 geförderten Blut B gebracht wird.

Zu Zwecken der Temperierung im Sinne einer gezielten Kühlung der den Wärmetauscher 3 durchströmenden Wärmeträgerflüssigkeit verfügt der Wärmetauscher 3 über Leitungsanschlüsse 6.1, 6.2, an die fluiddicht ein externer Schlauchleitungsabschnitt 8 ankoppelbar ist. Hierzu weist der Schlauchleitungsabschnitt 8 an seiner Zu- und Ableitungsöffnung 9, 10 jeweils einen geeignet ausgebildeten Schlauchleitungskonnektor, vorzugsweise in Form einer Luer-Lock-Verbindung oder einer Hansen-Kupplung auf. Die in Figur 1 dargestellten Strömungspfeile S1, S2 geben die Strömungsrichtung der Wärmeträgerflüssigkeit durch den geschlossenen Strömungskreislauf an, der sich aus dem Schlauchleitungssystem 4 innerhalb des Wärmetauschers 3 sowie dem externen Schlauchleitungsabschnitt 8 zusammensetzt, der Teil des Kühleinheit 1 ist.

Die Kühleinheit 1 weist im Wesentlichen zwei Behältnisse 11, 12 auf, die durch einen fluiddicht, membranartig ausgebildeten Kontaktabschnitt 13 voneinander separiert sind. Das erste der beiden Behältnisse 11 umfasst als flüssiges Arbeitsmittel Wasser (H₂O), typischerweise bemisst sich das Behältervolumen des ersten Behältnisses 11 zwischen 0,5 l und 2 l, vorzugsweise 1 l. Im zweiten, oberen Behältnis 12 ist ein Sorptionsmittel, vorzugsweise Zeolith eingebracht, das in der Lage ist, in die Dampfphase übergegangenes Wasser zu adsorbieren. Das zweite Behältnis 12 ist derart druckbeständig ausgebildet, so dass das Zeolith innerhalb des zweiten Behältnisses 12 unter Vakuumbedingungen bevorratet wird. Die Druckbedingungen innerhalb des ersten Behältnisses 11 sind vorzugsweise derart eingestellt, so dass das in Form von Wasser bevorratete flüssige Arbeitsmittel knapp unter dem Dampfdruck von Wasser liegt.

Mit Hilfe einer bspw. manuell betätigbaren Trennvorrichtung 14 lässt sich eine vorzugsweise lokal begrenzte Durchtrittsöffnung zu Zwecken einer fluidischen Verbindung zwischen dem ersten und zweiten Behältnis 11, 12 schaffen, wodurch Wasser in das zweite Behältnis 12 unter Bildung von Wasserdampf und Wärmeentzug aus dem im ersten Behältnis enthaltenen flüssigen Wassers entweicht. Durch den Verdampfungsprozess wird dem flüssigen Wasser innerhalb des ersten Behältnisses 11 Wärme entzogen, wodurch das Wasser signifikant abkühlt. Der innerhalb des ersten Behältnisses 11 verlaufende Schlauchleitungsabschnitt 8.1 tritt in unmittelbaren thermischen Kontakt mit dem sich abkühlenden bzw. abgekühlten Wasser, wodurch die längs des Schlauchleitungsabschnittes 8 geführte Wärmeträgerflüssigkeit gleichfalls abgekühlt wird. In dem gezeigten nicht erfindungsgemäßen Beispiel ist innerhalb des ersten Behältnisses 11 längs des Schlauchleitungsabschnittes 8.1 eine Förderpumpe 15 integriert, die für einen einstellbaren Strömungsfluss der Wärmeträgerflüssigkeit durch den Strömungskreislauf bestehend aus dem Schlauchleitungssystem 4 sowie dem Schlauchleitungsabschnittes 8 sorgt.

Zum Antrieb der Förderpumpe 15 dient eine Batterie, die bspw. am Ort der Förderpumpe innerhalb oder außerhalb des ersten Behälters angebracht ist. Gleichfalls ist es denkbar die Energiezufuhr zum Betrieb der Förderpumpe 15 im Wege einer induktiven Energieübertragung zu realisieren, so dass eine entsprechend elektromagnetische Energiequelle außerhalb des Behälters 1 angeordnet werden kann.

In vorteilhafter Weise sind das erste und zweite Behältnis 11, 12 aufgrund der in den jeweiligen Behälterbereichen entgegengesetzten Temperaturentwicklungen von einem thermisch isolierenden Gehäuse 16 umfasst.

Die lösungsgemäße Kühleinheit 1 vermag die durch den Wärmetauscher 3 geführte Wärmeträgerflüssigkeit für eine begrenzte Zeitdauer zu kühlen, die von dem Adsorptionsvermögen des innerhalb des zweiten Behältnisses 12 bevorrateten Sorptionsmittel Zeolith abhängt. Durch die kompakte, leichte und auch kostengünstige Ausgestaltung der Kühleinheit 1 ist diese vorzugsweise als Verbrauchsmaterial, im Sinne eines Einwegartikel ausgeführt. Bei entsprechender Bevorratung von zwei oder mehr lösungsgemäß ausgebildeter Kühleinheiten 1 kann eine in Betrieb befindliche Kühleinheit, deren Kühlleistung nachlässt, durch Auftrennen der Schlauchleitungskonnektoren 7.1, 7.2 durch eine neue, unverbrauchte Kühleinheit 1 ersetzt werden. Auf diese Weise lässt sich die Zeitdauer, während der Kühlleistung zur Verfügung gestellt werden muss, nahezu beliebig verlängern.

Zur Realisierung einer kühlleistungsoptimierten Kühleinheit kommt insbesondere der Abfuhr von Wärme, die im Wege der Verdampfung des flüssigen Arbeitsmittels in Form von Verdunstungswärme frei wird, besondere Bedeutung zu.

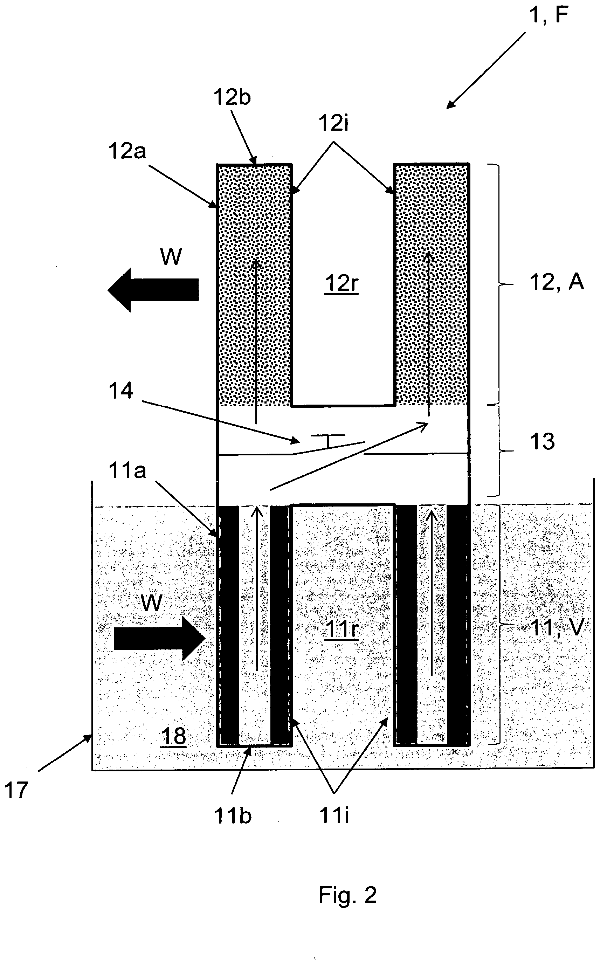

Fig. 2 zeigt einen schematisierten Aufbau einer erfindungsgemäßen kühlleistungsoptimierten Kühleinheit 1, die ein als Verdampfer V dienendes erstes Behältnis 11 sowie ein als Absorber A dienendes zweites Behältnis 12 umfasst, die beide über einen gemeinsamen fuiddichten Kontaktabschnitt 13 verbunden sind, längs dem ein vorzugsweise manuell bedienbares Trennmittel 14 eingebracht ist.

Das erste und zweite Behältnis 11, 12 sind jeweils als doppelwandig ausgebildeter Hohlzylinder ausgebildet, der jeweils über eine radial außen liegende Behältniswand 11a, 12a sowie eine radial innen liegende Behältniswand 11i, 12i verfügt, die gemeinsam mit einer jeweils stirnseitig orientierten, ringförmig ausgebildeten Behältniswand 11b, 12b ein innen liegendes Ringvolumen 11r, 12r umfassen. Beide Ringvolumina 11r, 12r sind hermetisch jeweils durch die doppelwandige Hohlzylinderwand sowie dem gemeinsamen fluiddichten Kontaktabschnitt 13 umschlossen.

Durch diese Behältnisgeometrie ist sowohl im Bereich des Verdampfers V als auch im Bereich des Absorbers A eine größtmögliche Behältniswandoberfläche geschaffen, über die Wärme W zu- bzw. abgeführt werden kann. Im Falle des als Verdampfer V dienenden ersten Behältnisses 11, das ein flüssiges Arbeitsmittel, vorzugsweise Wasser, gasdicht knapp unterhalb der Dampfdruckbedingungen des flüssigen Arbeitsmittels einschließt, - das Wasser ist vorzugsweise in einem Vlies innerhalb des ersten Behältnisses 11 bevorratet - erfolgt im Falle des Öffnens des manuell betätigbaren Trennmittels 14 und der damit einhergehenden Verdampfung des flüssigen Arbeitsmittels in das evakuierte Ringvolumen 12r des zweiten Behältnisses 12, das mit einem Sorptionsmittel, vorzugsweise Zeolith, gefüllt ist, und somit als Absorber A dient, eine Entwärmung des flüssigen Arbeitsmittels innerhalb des ersten Behälters 12 sowie eine Erwärmung des im zweiten Behälter 12 bevorrateten Sorptionsmittel, das das verdampfte Arbeitsmittel ab- oder adsorbiert Die innerhalb des ersten Behälters 11 erfolgende Entwärmung des flüssigen Arbeitsmittels wird technisch zur Abkühlung einer in einem Bad 17 bevorrateten Wärmeträgerflüssigkeit 18 genutzt. Hierzu taucht das erste Behältnis 11, vorzugsweise vollständig, in das aus der Wärmeträgerflüssigkeit, vorzugsweise ebenso Wasser, bestehende Bad 17 ein.

Die Behältniswände 11a, 11i, 11b, bzw. 12a, 12b, 12i sowohl des ersten als auch zweiten Behältnisses 11, 12 sind vorzugsweise jeweils einstückig aus metallischem und somit thermisch gut leitfähigem Material gefertigt, das darüber hinaus möglichst dünnwandig ausgebildet ist, vergleichbar jener von Getränkedosen. Ein bevorzugtes Herstellungsverfahren für die doppelwandig ausgebildeten Behältnisse stellt die sogenannte Fliesspresstechnik dar, insbesondere die Rückwärts-Fliesstechnik, die auch zur Herstellung von aus Aluminium gefertigten Getränkedosen Einsatz findet. Zu Seiten des als Absorber dienenden zweiten Behältnisses 12, in dem sich aufgrund der Absorption bzw. Adsorption des verdampften Arbeitsmittels das Sorptionsmittel signifikant erwärmt, gilt es, das Sorptionsmittel möglichst effektiv zu kühlen. Die Entwärmung des Sorptionsmittels erfolgt über eine möglichst große Oberfläche des zweiten Behälters 12, wie dies durch die Pfeildarstellung, die den Wärmefluss W illustrieren soll, gezeigt ist. Zur Unterstützung der Wärmeabführung über die Behältniswand des zweiten Behältnisses 12 dient, wie die weiteren Ausführungen zeigen werden, eine über die Behältniswandoberfläche gerichtete Kühlluftströmung, sowohl längs der radial außen liegenden als auch längs der radial innen liegenden Behältniswände 12a, 12i. Die Wärmeabführung im Bereich des Absorbers A ist entscheidend für die Entwärmung im Bereich des Verdampfers V. Durch die aktive Belüftung sowohl der radial innenliegenden als auch außenliegenden Hohlzylinderwand des Absorber A bzw. des zweiten Behältnisses 12 wird jeglicher Wärmestau vermieden.

Im Unterschied zu dem in Figur 1 illustrierten Beispiel, in dem zu Zwecken einer Entwärmung der Wärmeträgerflüssigkeit eine durch das erste Behältnis hindurch führende Schlauchleitung vorgesehen ist, taucht in Falle des in Fig. 2 illustrierten Beispiels das erste Behältnis 11 vorzugsweise vollständig in das mit der Wärmeträgerflüssigkeit gefüllte Bad 17 ein. Dies eröffnet die Möglichkeit eine aus dem ersten Behältnis 11, dem zweiten Behältnis 12 sowie dem beide Behältnisse 11, 12 miteinander fest verbindenden gemeinsamen Kontaktbereich 13 bestehende, einheitlich handhabbare kühlende Funktionseinheit F modulartig aus dem Bad 17 zu entnehmen und durch eine andere, ansonsten identisch ausgebildete Funktionseinheit zu ersetzen. Auf diese Weise lässt sich die Kühlung der im Bad 17 enthaltenen Wärmeträgerflüssigkeit 18 thermisch sowie auch zeitlich nahezu beliebig vornehmen bzw. ausdehnen.

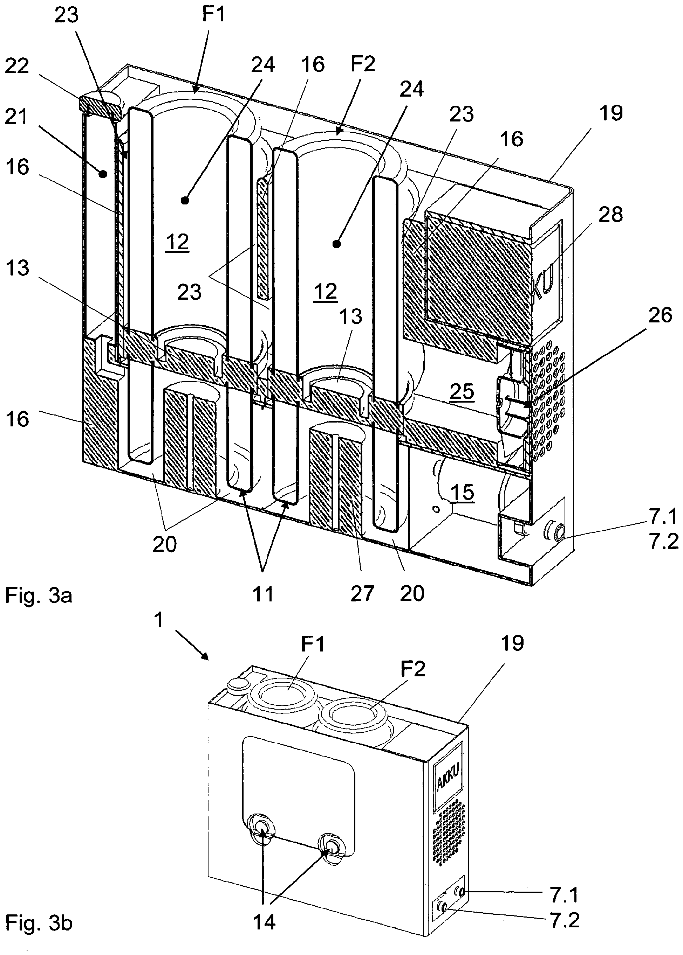

Auf Basis der in Figur 2 illustrierten kühlenden Funktionseinheit F lässt sich ein konkretes Ausführungsbeispiel für eine Kühleinheit realisieren, die in Figur 3a in einer Schnittzeichnung und in Figur 3b in einer Gesamtansicht gezeigt ist.

Figur 3b zeigt die Außenansicht einer lösungsgemäß ausgebildeten Kühleinheit 1, die von einem portablen Gehäuse 19 umfasst ist, das nicht größer als das Volumen einer typischen Aktentasche, maximal eines Koffers, ist sowie ein Gewicht von maximal 25 kg, vorzugsweise kleiner oder gleich 15 kg besitzt. Am Gehäuse 19 der Kühleinheit 1 sind Schlauchleitungskonnektoren 7.1, 7.2 zum lösbar fluiddichten Anschluss eines externes Schlauchleitungssystem angebracht, durch das die Wärmeträgerflüssigkeit eines in einem Oxygenator integrierten Wärmetauschers geführt ist. Die Kühleinheit 1 ist derart konfektioniert, so dass eine durch die Kühleinheit strömende Wärmeträgerflüssigkeit mit einem Fördervolumen von 3 bis 8 Liter pro Minute, vorzugsweise 4 l/min abgekühlt und einem in einem Oxygenator integrierten Wärmetauschers zur Abkühlung von durch den Oxygenator hindurch strömenden Patientenblutes zugeführt wird, so dass das Patientenblut innerhalb von 30 Minuten um wenigstens 4 Kelvin abgekühlt wird. Dies spiegelt die Leistungsfähigkeit der lösungsgemäßen Kühleinheit 1 wieder.

Zum besseren Verständnis des Aufbaus der lösungsgemäß ausgebildeten Kühleinheit 1 wird neben der die Kühleinheit 1 illustrierenden Gesamtansicht gemäß Figur 3b zusätzlich auf die Schnittdarstellung gemäß Figur 3a verwiesen.

Im unteren Teil des Gehäuses 19 der Kühleinheit 1 befindet sich eine nach oben offene Kammer 20, durch die die Wärmeträgerflüssigkeit 18 geleitet wird. Die Durchleitung der Wärmeflüssigkeit erfolgt pumpengetrieben. Hierzu befindet sich innerhalb des Gehäuses 10 eine Förderpumpe 15, die einerseits mit dem Schlauchleitungskonnektor 7.1.und andererseits mit der Kammer 20 fluidisch verbunden ist. Der andere Schlauchleitungskonnektor 7.2 ist (nicht dargestellt) über eine Schlauchleitung direkt mit der Kammer 20 fluidisch verbunden.

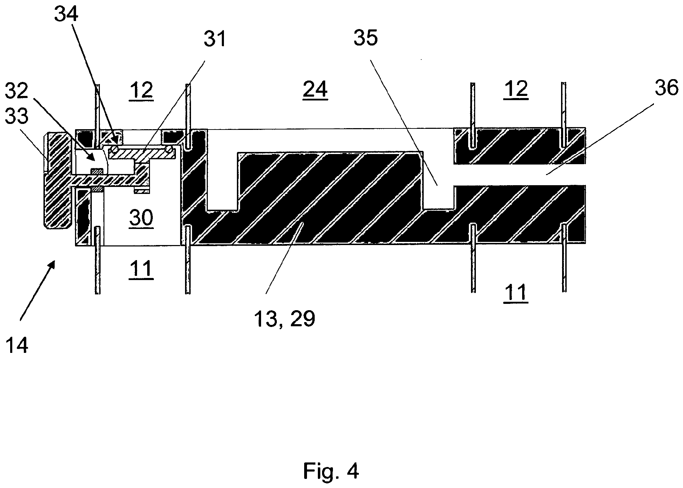

Die Kammer 20 ist ferner mit einem Vorratstank bzw. Befüllungstank 21 fluidisch verbunden, in dem Wärmeträgerflüssigkeit über eine entsprechende verschließbare Öffnung 22 einfüllbar ist. Die Kammer 20 ist in Form und Größe zur Aufnahme von zwei einzelnen, modular handhabbaren kühlenden Funktionseinheiten F1, F2 ausgebildet. Die baugleich ausgebildeten kühlenden Funktionseinheiten F1, F2 weisen jeweils den Figur 2 schematisiert dargestellten Aufbau auf. So ragt das jeweils erste Behältnis 11 der Funktionseinheiten F1, F2 vollständig von oben in die Kammer 20 und vermag diese mit Hilfe des jeweils gemeinsamen Kontaktabschnittes 13 fluiddicht abzuschließen. Der jeweils gemeinsame Kontaktabschnitt 13 ist als ringförmig ausgebildetes Plattenelement ausgeformt und dient zum einen der mechanischen Verbindung des jeweils ersten und zweiten Behältnisses 11, 12 zum anderen der Integration eines manuell bedienbaren Trennmittels 14, das in Figur 3a nicht dargestellt ist. Figur 3b zeigt lediglich den manuell bedienbaren Drehknopf des Trennmittels 14. Zur diesbezüglichen Erläuterung wird im Weiteren auf Figur 4 verwiesen.

Sowohl zu Zwecken eines mechanisch sicheren Sitzes als auch eines fluiddichten Abschlusses der in die Kühleinheit eingesetzten, kühlenden Funktionseinheit F1, F2 mit der Kammer 20, ist im Bereich des gemeinsamen Kontaktabschnitt 13 eine geeignet ausgebildete, dichtende Befestigungsstruktur vorgesehen, bspw. in Form eines Bajonettverschlusses oder eine Presssitzes.

Das als Absorber dienende zweite Behältnis 12, das sich in Figur 3a vertikal über den gemeinsamen Kontaktabschnitt 13 erstreckt, ist gleichsam wie das erste Behältnis 11 von einer thermisch isolierenden Umfassung 16 innerhalb des Gehäuses 19 umgeben, die jeweils mit der radial außen liegenden Behältniswand 12a pro kühlender Funktionseinheit F1, F2 einen ringförmigen Zwischenspalt 23 einschließt, der nach oben offen endet. Gleichsam umfasst das jeweils zweite Behältnis 12 ein nach oben hin offen ausgebildetes inneres Hohlzylindervolumen 24. Sowohl die ringförmigen Zwischenspalte 23 sowie auch die inneren Hohlzylindervolumina 24 beider kühlenden Funktionseinheiten F1, F2 sind fluidisch mit einem innerhalb des Gehäuses 19 vorgesehenen Strömungskanal 25 verbunden, in dem Ventilatoreinheit 26 vorgesehen ist. Auf diese Weise wird sichergestellt, dass eine durch die Ventilatoreinheit 26 initiierte Kühlluftströmung sowohl axial längs der ringförmigen Zwischenspalte 23 als auch längs der inneren Hohlzylindervolumina 24, vorzugsweise mit einer vertikal von unten nach oben orientierten Durchströmungsrichtung angelegt werden kann. Hierdurch ist eine effiziente Kühlung der jeweils als Absorber dienenden zweiten Behältnisse 12 der modularen Funktionseinheiten F1 und F2 gewährleistet.

Um den Wärmeaustauch zwischen den jeweils ersten Behältnissen 11 und der in der Kammer 20 bevorrateten Wärmeträgerflüssigkeit zu optimieren, ist das Kammervolumen 20 durch zylinderförmig ausgebildete Verdrängungskörper 27, die in das innere Hohlzylindervolumen des jeweils ersten Behältnisses 11 hinein ragen, reduziert. Auf diese Weise kann sichergestellt werden, dass die in der Kammer 20 befindliche Wärmeträgerflüssigkeit in einen effizienten Wärmeaustausch mit den Behältniswänden der jeweils ersten Behältnisse 11 gelangt.

Zusätzlich innerhalb des Gehäuses 19 ist eine Stromversorgungseinheit 28 untergebracht, die zur elektrischen Energieversorgung sowohl der Förderpumpe 15 als auch der Ventilatoreinheit 26 dient. Die Energieversorgungseinheit 28 ist vorzugsweise in Form einer Batterie oder eines an ein Stromnetz anschließbaren Netzteils ausgebildet.

Die lösungsgemäß ausgebildete Kühleinheit 1 sieht im dargestellten Ausführungsbeispiel zwei modular aus dem Gehäuse 19 entnehmbare bzw. einsetzbare kühlende Funktionseinheiten F1 und F2 vor, die eine kontinuierliche Kühlung der durch die Kammer 20 strömenden Wärmeträgerflüssigkeit ermöglicht. In einer bevorzugten Ausführungsform umfasst jedes der beiden modulartig ausgebildeten Funktionseinheiten F1 und F2 jeweils etwa 200 g Wasser als flüssiges Arbeitsmittel innerhalb des ersten Behältnisses, das von einem Vlies aufgenommen ist, sowie ca. 1 kg Zeolith als Sorptionsmittel innerhalb des zweiten Behältnisses.

Lässt die Kühlwirkung der jeweils als, vorzugsweise Einwegartikel ausgebildeten kühlenden Funktionseinheiten F1, F2 nach, indem das Sorptionsmittel das im jeweils ersten Behältnis bevorratete flüssig Arbeitsmittel vollständig ab- oder adsorbiert hat, so kann für eine weitere Aufrechterhaltung der Kühlleistung eine verbrauchte Funktionseinheit gegen eine unverbrauchte Funktionseinheit ersetzt werden.

In Figur 4 ist eine Längsschnittdarstellung durch eine konkrete Ausbildungsform eines fluiddichten Kontaktabschnittes 13 gezeigt, der das erste und zweite Behältnis 11, 12 miteinander mechanisch fest und fluiddicht verbindet. Der fluiddichte Kontaktabschnitt 13 ist vorzugsweise in Form eines scheibenförmig ausgebildeten Verbindungselementes 29 ausgebildet, das gleichsam wie die Behältniswände des ersten und zweiten Behältnisses 11, 12 aus einem metallischen, vorzugsweise aus Aluminium gefertigt ist. Gemäß Figur 4 münden die jeweils stirnseitig einseitig endenden Behältniswände des ersten und zweiten Behältnisses 11, 12 einseitig fluiddicht an bzw. in dem scheibenförmigen Verbindungselement 29.

Für eine kontrollierte Verbindung der Volumina des ersten und zweiten Behältnisses 11, 12 ist in dem scheibenförmigen Verbindungselement 29 lokal ein Trennmittel 14 integriert, das längs eines Verbindungskanals 30 eine fluiddicht abschließende Abdeckplatte 31 vorsieht, die über eine Exzentermechanik 32 durch ein manuelles Betätigungsmittel 33 axial längs des Verbindungskanals 30 beweglich gelagert ist und ein Öffnen sowie auch Verschließen des Verbindungskanals 30 ermöglicht. In einer Ausgangssituation ist das Volumen des zweiten Behältnisses 12 evakuiert, wohingegen im Volumen des ersten Behältnisses ein dem Dampfdruck des flüssigen Arbeitsmittels, vorzugsweise Wasser, entsprechender Druck vorherrscht. Durch das herrschende Druckgefälle wird die Abdeckplatte 31 selbstsichernd und fluiddicht gegen eine entsprechende Kanalkulisse 34 gepresst, so dass keine weiteren Maßnahmen erforderlich sind, eine zu bevorratende Funktionseinheit F1, F2 vor unkontrolliertem Verdampfen des flüssigen Arbeitsmittels zu schützen.

An der Oberseite des scheibenförmigen Verbindungselementes 29 ist im Bereich des inneren Hohlzylindervolumens 24 eine nutförmige Ausnehmung 35 eingebracht, in die ein seitlicher Verbindungskanal 36 mündet, durch den eine künstliche Luftströmung zur Kühlung der radialen Innenwand des zweiten Behältnisses 12 in das innere Hohlzylindervolumen 24 einströmen kann. Der Verbindungskanal 36 mündet im Bereich des Strömungskanals 25 innerhalb des Gehäuses 19.

Selbstverständlich ist es möglich, zu Zwecken der Kühlleistungssteigerung die vorstehend illustrierte Kühleinheit 1 mit mehr als zwei modular ausgebildeten kühlenden Funktionseinheiten F1 und F2 bestückbar auszubilden, sofern entsprechende Kühlleistung gewünscht ist und eine dadurch bedingte Gewichts- und Volumenzunahme der lösungsgemäß ausgebildeten Kühleinheit tolerabel erscheint.

### Bezugszeichenliste

- 1: Kühleinheit
- 2: Oxygenator
- 3: Wärmetauscher
- 4: Schlauchleitungssystem
- 5: extrakorporaler Blutkreislauf
- 6.1, 6.2: Leitungsanschlüsse
- 7.1, 7.2: Schlauchleitungskonnektoren
- 8: Schlauchleitungsabschnitt
- 8.1: Schlauchleitungsabschnitt innerhalb des ersten Behälters
- 8.2: Schlauchleitungsabschnitt außerhalb des ersten Behälters
- 9: Zuleitungsöffnung
- 10: Ableitungsöffnung
- 11: erster Behälter
- 12: zweiter Behälter
- 13: fluiddichter Kontaktabschnitt
- 14: Trennmittel
- 15: Förderpumpe
- 16: thermisch isolierende Umfassung, Gehäuse
- 17: Bad
- 18: Wärmeträgerflüssigkeit
- 19: Gehäuse
- 20: Kammer
- 21: Vorratstank
- 22: Verschließbare Öffnung
- 23: Ringförmiger Zwischenspalt
- 24: Inneres Hohlzylindervolumen
- 25: Strömungskanal
- 26: Ventilatoreinheit
- 27: Verdrängungskörper
- 28: Energieversorgungseinheit
- 29: Scheibenförmiges Verbindungselement
- 30: Verbindungskanal
- 31: Fluiddichte Abdeckung
- 32: Exzentermechanik
- 33: Betätigungsmittel
- 34: Kanalkulisse
- 35: Nutförmige Ausnehmung
- 36: Verbindungskanal

## Patentansprüche

1. Kühleinheit (1) für einen in einem Oxygenator (2) integrierten Wärmetauscher (3) zur Temperierung von in einem extrakorporalen Blutkreislauf (5) geführtem Blut, mit einem ersten Behältnis (11), das zumindest teilweise mit einem flüssigen Arbeitsmittel befüllt ist, das in thermischen Kontakt mit einer Wärmeträgerflüssigkeit des Wärmetauschers (3) bringbar ist, wobeiein zweites Behältnis (12) vorgesehen ist, das ein Sorptionsmittel unter Vakuumbedingungen fluiddicht umschließt und über wenigstens einen Kontaktabschnitt (13) an das erste Behältnis (11) unmittelbar angrenzt, wobei im Kontaktabschnitt (13) ein betätigbares Trennmittel (14) angebracht ist, das bei Betätigung eine lokal begrenzte fluidische Verbindung zwischen dem ersten (11) zum zweiten Behältnis (12) herstellt, wodurch das flüssige Arbeitsmittel in das zweite Behältnis (12) unter Bildung eines Arbeitsmitteldampfes und Wärmeentzug aus dem im ersten Behältnis (11) enthaltenen flüssigen Arbeitsmittel entweicht, wobei das erste und zweite Behältnis (11, 12) von einer thermisch isolierenden Umfassung (16) umgeben sind, wobei das erste und zweite Behältnis (11, 12) zusammen mit dem Kontaktabschnitt (13) jeweils ein Volumen hermetisch umschliessen, und das erste Behältnis (11) wenigstens teilweise in ein Bad (17) mit der Wärmeträgerflüssigkeit eintaucht,
**dadurch gekennzeichnet, dass** das erste und zweite Behältnis (11, 12) jeweils als doppelwandige Hohlzylinder ausgebildet sind, die jeweils ein Ringvolumen (11r, 12r) umschließen, und
dass beide doppelwandig ausgebildete Hohlzylinder längs ihrer den beiden Hohlzylindern zugeordneten Zylinderachsen einander stirnseitig zugewandt über den gemeinsamen Kontaktabschnitt (13) verbunden sind und eine modulare, einheitlich handhabbare kühlende Funktionseinheit bilden.

2. Kühleinheit nach Anspruch 1,
**dadurch gekennzeichnet, dass** das Sorptionsmittel synthetisches Zeolith und/oder natürliches Zeolith enthält.

3. Kühleinheit nach Anspruch 1 oder 2,
**dadurch gekennzeichnet, dass** das erste und zweite Behältnis (11, 12) aus der thermischen Umfassung (16) modular entnehmbar und in die thermische Umfassung modular einsetzbar sind.

4. Kühleinheit nach einem der Ansprüche 1 bis 3,
**dadurch gekennzeichnet, dass** das erste Behältnis (11) ein Volumen von 0,5 l bis 2 l umfasst und Wasser als Arbeitsmittel bei Druckbedingungen einschließt, die knapp unter dem Dampfdruck von Wasser liegen.

5. Kühleinheit nach Anspruch 3,
**dadurch gekennzeichnet, dass** das Bad (17) mit der Wärmeträgerflüssigkeit in der thermischen Umfassung (16) bevorratet ist, in das das erste Behältnis (11) entnehmbar einsetzbar ist.

6. Kühleinheit nach Anspruch 1,
**dadurch gekennzeichnet, dass** die doppelwandigen Hohlzylinder jeweils eine radial äußere (11a, 12a) und eine radial innere Behältniswand (11i, 12i) aufweisen,
dass die radial innere Behältniswand (11i, 12i) ein inneres Hohlzylindervolumen (11r, 12r) radial begrenzt, das jeweils dem gemeinsamen Kontaktabschnitt (13) axial abgewandt offen zugänglich ist,
dass die thermische Umfassung (16) eine mit dem flüssigen Arbeitsmittel befüllbare Kammer (20) aufweist, die zur Aufnahme des ersten Behältnisses (11) derart ausgebildet ist, dass das erste Behältnis (11) die Kammer (20) fluiddicht abschliesst und die radial äußere und innere Behältniswand (11a, 11i) des ersten Behältnisses (11) thermisch mit dem flüssigen Arbeitsmittel in Kontakt bringbar sind, und
dass die thermische Umfassung (16) das zweite Behältnis (12) axial vollständig umfasst und mit der radial äußeren Behältniswand (12a) des zweiten Behältnisses (12) einen radial, ringförmigen Belüftungspalt (23) einschließt, der gemeinsam mit dem inneren Hohlzylindervolumen (24) des zweiten Behältnisses (12) axial stirnseitig offen mündet.

7. Kühleinheit nach Anspruch 6,
**dadurch gekennzeichnet, dass** sowohl der ringförmige Belüftungspalt (23) als auch das innere Hohlzylindervolumen (24) des zweiten Behältnisses (12) mit einem Belüftungskanal (25) verbunden sind, längs dem eine Ventilatoreinheit (26) eingebracht ist.

8. Kühleinheit nach einem der Ansprüche 1 bis 7,
**dadurch gekennzeichnet, dass** die thermische Umfassung (16) Teil eines portablen Gehäuses (19) ist,
dass innerhalb des Gehäuses (19) und außerhalb der thermischen Umfassung (16) eine Förderpumpe (15) vorgesehen ist, die über eine Zu- und Ableitung fluidisch mit dem Bad (17) aus der Wärmeträgerflüssigkeit verbunden ist, und
dass am Gehäuse (19) mit der Zu- und Ableitung fluidisch verbundene Schlauchleitungskonnektoren angebracht sind, an die ein Schlauchleitungssystem des Wärmetauschers fluidisch anschließbar ist.

9. Kühleinheit nach Anspruch 8,
**dadurch gekennzeichnet, dass** innerhalb des Gehäuses (19) und außerhalb der thermischen Umfassung (16) die Ventilatoreinheit (26) sowie eine elektrische Energieversorgungseinheit (28) vorgesehen sind.

10. Kühleinheit nach Anspruch 1 sowie 8 oder 9,
**dadurch gekennzeichnet, dass** das portable Gehäuse (19) sowie die thermische Umfassung (16) zur modularen Aufnahme von wenigstens zwei Funktionseinheiten parallel nebeneinander ausgebildet ist.

11. Kühleinheit nach einem der Ansprüche 8 bis 10,
**dadurch gekennzeichnet, dass** die Förderpumpe (15) eine Förderleistung von 2 bis 8 l/min, vorzugsweise 4 l/min besitzt.

12. Kühleinheit nach einem der Ansprüche 1 bis 11,
**dadurch gekennzeichnet, dass** die wenigstens eine kühlende Funktionseinheit als modularer Einwegartikel ausgebildet ist.

## Claims

1. A cooling unit (1) for a heat exchanger (3) integrated in an oxygenator (2) for controlling the temperature of blood conducted in an extracorporeal blood circuit (5), comprising a first container (11) which is at least partially filled with a liquid working medium which is able to be brought into thermal contact with a heat transfer fluid of the heat exchanger (3), wherein a second container (12) is provided, said second container enclosing a sorbent in a fluid-tight manner and under vacuum conditions and directly adjoining the first container (11) via at least one contact portion (13), wherein an actuatable release means (14) is installed in the contact portion (13), which release means, when actuated, generates a locally restricted fluidic connection between the first (11) and second containers (12), whereby the liquid working medium escapes into the second container (12) forming a working medium vapour and extracting heat from the liquid working medium contained in the first container (11), wherein the first and second containers (11, 12) are surrounded by a thermally insulating enclosure (16), wherein the first and second containers (11, 12) together with the contact portion (13) in each case enclose a volume in a hermetically sealed manner, and the first container (11) is at least partially immersed in a bath (17) comprising the heat transfer fluid,
**characterised in that** the first and second containers (11, 12) are configured in each case as double-walled hollow cylinders which respectively enclose an annular volume (11r, 12r) and
**in that** the two hollow cylinders which are of double-walled configuration face one another on the front face and are connected together along their cylinder axes assigned to the two hollow cylinders via the common contact portion (13) and form a modular cooling functional unit which is able to be handled as a single unit.

2. The cooling unit according to Claim 1, **characterised in that** the sorbent contains synthetic zeolite and/or natural zeolite.

3. The cooling unit according to Claim 1 or 2, **characterised in that** the first and second containers (11, 12) are able to be removed in a modular manner from the thermal enclosure (16) and are able to be inserted in a modular manner into the thermal enclosure.

4. The cooling unit according to one of Claims 1 to 3, **characterised in that** the first container (11) encloses a volume of 0.5 l to 2 l and comprises water as a working medium at pressure conditions which are just below the vapour pressure of water.

5. The cooling unit according to Claim 3, **characterised in that** the bath (17) comprising the heat transfer fluid is stored in the thermal enclosure (16), the first container (11) being able to be removably inserted therein.

6. The cooling unit according to Claim 1, **characterised in that** the double-walled hollow cylinders in each case have a radial external container wall (11a, 12a) and a radial internal container wall (11i , 12i), **in that** the radial internal container wall (11i, 12i) radially defines an internal hollow cylinder volume (11r, 12r) which in each case is open so as to be accessible axially remote from the common contact portion (13), **in that** the thermal enclosure (16) has a chamber (20) which is able to be filled by the liquid working medium and which is configured for receiving the first container (11) such that the first container (11) seals the chamber (20) in a fluid-tight manner and the radial external and internal container walls (11a, 11i) of the first container (11) are able to be brought into thermal contact with the liquid working medium, and **in that** the thermal enclosure (16) fully encloses the second container (12) axially and with the radial external container wall (12a) of the second container (12) encompasses a radial annular ventilation gap (23) which together with the internal hollow cylinder volume (24) of the second container (12) is open axially on the front face.

7. The cooling unit according to Claim 6, **characterised in that** both the annular ventilation gap (23) and the internal hollow cylinder volume (24) of the second container (12) are connected to a ventilation channel (25), a ventilator unit (26) being incorporated along said ventilation channel.

8. The cooling unit according to one of Claims 1 to 7, **characterised in that** the thermal enclosure (16) is part of a portable housing (19), **in that** a delivery pump (15) which is fluidically connected via a supply and discharge line to the bath (17) consisting of the heat transfer fluid is provided inside the housing (19) and outside the thermal enclosure (16), and **in that** hose pipe connectors which are fluidically connected to the supply and discharge line are attached to the housing (19), a hose pipe system of the heat exchanger being able to be fluidically connected thereto.

9. The cooling unit according to Claim 8, **characterised in that** the ventilator unit (26) and an electrical energy supply unit (28) are provided inside the housing (19) and outside the thermal enclosure (16).

10. The cooling unit according to Claim 1 and 8 or 9, **characterised in that** the portable housing (19) and the thermal enclosure (16) are configured for receiving in a modular manner at least two functional units in parallel and adjacent to one another.

11. The cooling unit according to one of Claims 8 to 10, **characterised in that** the delivery pump (15) has a delivery output of 2 to 8 l/min, preferably 4 l/min.

12. The cooling unit according to one of Claims 1 to 11, **characterised in that** the at least one cooling functional unit is configured as a modular disposable article.

## Revendications

1. Unité de refroidissement (1) pour un échangeur de chaleur (3) intégré dans un oxygénateur (2) pour tempérer du sang circulant dans un circuit sanguin extracorporel (5), avec un premier contenant (11) au moins partiellement rempli avec un fluide de travail liquide qui peut être mis en contact thermique avec un liquide caloporteur de l'échangeur de chaleur (3), dans laquelle un deuxième contenant (12) est prévu, lequel enferme de manière étanche aux fluides dans des conditions sous vide un adsorbant et est directement adjacent au premier contenant (11) via au moins une partie de contact (13), dans laquelle un moyen de séparation actionnable (14) est monté dans la partie de contact (13), lequel, en cas d'actionnement, établit une liaison fluidique limitée localement entre le premier (11) et le deuxième contenant (12), moyennant quoi le fluide de travail liquide s'échappe dans le deuxième contenant (12) en formant une vapeur de fluide de travail et en éliminant de la chaleur du fluide de travail liquide contenu dans le premier contenant (11), dans laquelle le premier et le deuxième contenant (11, 12) sont entourés par un enveloppement d'isolation thermique (16), dans laquelle le premier et le deuxième contenant (11, 12) enferment hermétiquement, ensemble avec la partie de contact (13), respectivement un volume, et le premier contenant (11) plonge au moins partiellement dans un bain (17) avec le liquide caloporteur,
**caractérisée en ce que** le premier et le deuxième contenant (11, 12) sont respectivement réalisés en tant que cylindres creux à double paroi qui enferment respectivement un volume annulaire (11r, 12r), et
que les deux cylindres creux réalisés à double paroi sont reliés, le long de leurs axes de cylindre associés aux deux cylindres creux, de manière tournée l'un vers l'autre du côté frontal via la partie de contact (13) commune, et forment une unité fonctionnelle de refroidissement modulaire pouvant être manipulée de façon homogène.

2. Unité de refroidissement selon la revendication 1, **caractérisée en ce que** l'adsorbant contient de la zéolithe synthétique et/ou de la zéolithe naturelle.

3. Unité de refroidissement selon la revendication 1 ou 2, **caractérisée en ce que** le premier et le deuxième contenant (11, 12) peuvent être retirés de façon modulaire de l'enveloppement thermique (16) et être mis en place de façon modulaire dans l'enveloppement thermique.

4. Unité de refroidissement selon l'une des revendications 1 à 3, **caractérisée en ce que** le premier contenant (11) comprend un volume de 0,5 l à 2 l et englobe de l'eau en tant que fluide de travail avec des conditions de pression qui se situent juste sous la pression de vapeur de l'eau.

5. Unité de refroidissement selon la revendication 3, **caractérisée en ce que** le bain (17) avec le liquide caloporteur est stocké dans l'enveloppement thermique (16) dans lequel le premier contenant (11) peut être mis en place de façon à pouvoir être retiré.

6. Unité de refroidissement selon la revendication 1, **caractérisée en ce que** les cylindres creux à double paroi présentent respectivement une paroi de contenant radialement extérieure (11a, 12a) et une paroi de contenant radialement intérieure (11i, 12i), que la paroi de contenant radialement intérieure (11i, 12i) délimite radialement un volume intérieur de cylindre creux (11r, 12r) qui est respectivement ouvertement accessible en tournant le dos axialement à la partie de contact (13) commune,
que l'enveloppement thermique (16) présente une chambre (20) pouvant être remplie avec le fluide de travail liquide, laquelle est réalisée de manière à ce que, pour la réception du premier contenant (11), le premier contenant (11) ferme la chambre (20) de manière étanche aux fluides et que les parois de contenant radialement extérieure et intérieure (11a, 11i) du premier contenant (11) peuvent être mises en contact thermique avec le fluide de travail liquide, et
que l'enveloppement thermique (16) entoure complètement axialement le deuxième contenant (12) et entoure, avec la paroi de contenant radialement extérieure (12a) du deuxième contenant (12), une fente d'aération annulaire radiale (23) qui débouche axialement de manière ouverte du côté frontal ensemble avec le volume intérieur de cylindre creux (24) du deuxième contenant (12).

7. Unité de refroidissement selon la revendication 6, **caractérisée en ce que** la fente d'aération (23) de forme annulaire tout comme également le volume intérieur de cylindre creux (24) du deuxième contenant (12) sont reliés à un canal d'aération (25) le long duquel une unité de ventilateur (26) est intégrée.

8. Unité de refroidissement selon l'une des revendications 1 à 7, **caractérisée en ce que** l'enveloppement thermique (16) est une partie d'un boîtier portatif (19),
qu'à l'intérieur du boîtier (19) et à l'extérieur de l'enveloppement thermique (16), une pompe d'alimentation (15) est prévue, laquelle est reliée de manière fluidique via une amenée et une évacuation au bain (17) de liquide caloporteur, et
**en ce qu'**au niveau du boîtier (19), des raccords de tuyaux flexibles reliés de manière fluidique à l'amenée et l'évacuation sont mis en place, auxquels il est possible de raccorder de manière fluidique un système de tuyaux flexibles de l'échangeur de chaleur.

9. Unité de refroidissement selon la revendication 8, **caractérisée en ce qu'**à l'intérieur du boîtier (19) et à l'extérieur de l'enveloppement thermique (16) on prévoit l'unité de ventilateur (26) ainsi qu'une unité d'alimentation électrique (28).

10. Unité de refroidissement selon la revendication 1 ainsi que 8 ou 9, **caractérisée en ce que** le boîtier portatif (19) ainsi que l'enveloppement thermique (16) sont réalisés pour la réception modulaire d'au moins deux unités fonctionnelles parallèlement côte à côte.

11. Unité de refroidissement selon l'une des revendications 8 à 10, **caractérisée en ce que** la pompe d'alimentation (15) possède un débit de 2 à 8 l/min, de préférence de 4 l/min.

12. Unité de refroidissement selon l'une des revendications 1 à 11, **caractérisée en ce que** l'au moins une unité fonctionnelle de refroidissement est réalisée en tant qu'article modulaire à usage unique.
